# EUROPEAN PATENT SPECIFICATION

(11) **EP 3 030 231 B1**
(45) Date of publication and mention of the grant of the patent: **23.06.2021**
(21) Application number: 14750206.6
(22) Date of filing: 07.08.2014
(51) Int. Cl.: A61K 31/215, A61K 31/22, A61K 31/225, A61P 21/06

(54) **KETONE BODY AND KETONE BODY ESTER FOR REDUCING MUSCLE BREAKDOWN**
KETONKÖRPER UND KETONKÖRPERESTER ZUR VERRINGERUNG VON MUSKELABBAU
CORPS CÉTONIQUE ET ESTER DE CORPS CÉTONIQUE POUR RÉDUIRE LA DÉGRADATION MUSCULAIRE

(30) Priority: 07.08.2013 GB 201314127
(43) Date of publication of application: 15.06.2016
(73) Proprietor: Tdeltas Limited, Thame OX9 3EZ (GB)
(72) Inventor: CLARKE, Kieran, Oxford Oxfordshire OX1 3PT (GB); COX, Peter, Oxford OxfordshireOX1 3PT (GB)
(74) Representative: Geary, Stephen
(86) International application number: PCT/EP2014/067027
(87) International publication number: WO 2015/018913

(56) References cited:
- WO-A1-2011/101171
- WO-A2-2004/108740
- WU G ET AL: "Ketone bodies inhibit leucine degradation in chick skeletal muscle", INTERNATIONAL JOURNAL OF BIOCHEMISTRY, PERGAMON, vol. 19, no. 10, 1 January 1987 (1987-01-01), pages 937-943, XP023403024, ISSN: 0020-711X, DOI: 10.1016/0020-711X(87)90175-3 [retrieved on 1987-01-01]
- SHERWIN R S ET AL: "Effect of ketone infusions on amino acid and nitrogen metabolism in man.", THE JOURNAL OF CLINICAL INVESTIGATION JUN 1975, vol. 55, no. 6, June 1975 (1975-06), pages 1382-1390, XP002731049, ISSN: 0021-9738
- NAIR K S ET AL: "Effect of beta-hydroxybutyrate on whole-body leucine kinetics and fractional mixed skeletal muscle protein synthesis in humans.", THE JOURNAL OF CLINICAL INVESTIGATION JUL 1988, vol. 82, no. 1, July 1988 (1988-07), pages 198-205, XP002731050, ISSN: 0021-9738
- SMITH R ET AL: "Initial effect of injury on ketone bodies and other blood metabolites.", LANCET 4 JAN 1975, vol. 1, no. 7897, 4 January 1975 (1975-01-04), pages 1-3, XP002731051, ISSN: 0140-6736
- FELIG P ET AL: "Amino acid metabolism in exercising man.", THE JOURNAL OF CLINICAL INVESTIGATION DEC 1971, vol. 50, no. 12, December 1971 (1971-12), pages 2703-2714, XP002731052, ISSN: 0021-9738
- LAFFEL L: "Ketone bodies: a review of physiology, pathophysiology and application of monitoring to diabetes.", DIABETES/METABOLISM RESEARCH AND REVIEWS, vol. 15, no. 6, November 1999 (1999-11), pages 412-426, ISSN: 1520-7552
- KIERAN CLARKE ET AL: "Oral 28-day and developmental toxicity studies of ()-3-hydroxybutyl ()-3-hydroxybutyrate", REGULATORY TOXICOLOGY AND PHARMACOLOGY, vol. 63, no. 2 11 April 2012 (2012-04-11), pages 196-208, XP028517092, ISSN: 0273-2300, DOI: 10.1016/J.YRTPH.2012.04.001 [retrieved on 2012-04-11]

## Description

This invention relates to the use of an ester of D-β-hydroxybutyrate for improving endurance during exercise and muscle recovery after exercise.

Substrate metabolism in the normal human body utilises different fuel sources depending on their availability. During exercise, energy expenditure increases dramatically above resting levels, with rapid mobilisation of fuels required to keep pace with ATP demand. Usually, as exercise intensity increases, mitochondrial oxidation of fatty acids reaches a ceiling, shifting the burden of energy provision to carbohydrates, so that glycolytic supply of pyruvate is the major carbon source for oxidation during heavy exercise.

In the fed state, muscle uses glucose and fatty acids for energy metabolism. However, during prolonged or intense exercise, protein from the break-down of muscle plays an important role in providing glucose. As muscle is broken down, levels of the essential branched chain amino acids, leucine, isoleucine and valine, increase and provide an indication of the extent of muscle breakdown.

Along with fats and carbohydrates, ketone bodies are metabolised by the body for energy. Ketone bodies are produced in the liver when fatty acids are released from body fat during a fast or in starvation. Raising ketone body concentrations in the blood, for example using a ketogenic diet, can have various clinical benefits, including treatment of epilepsy, diabetes and Parkinson's disease.

WO2004/108740 discloses compounds and compositions containing D-β-hydroxybutyrate derivatives effective for elevating blood concentrations of ketone bodies and methods for using such compounds, particularly oligomers and compositions, as nutritional supplements or for treating medical conditions. D-β-hydroxybutyrate derivatives and compositions that include these derivatives may serve as precursors to the ketone bodies, acetoacetate and D-β-hydroxybutyrate, and are said to elevate blood concentrations of ketone bodies when administered to a subject.

WO2004/105742 discloses the use of a compound, for example ketone bodies, which reduce free fatty acids circulating in the blood plasma of a subject for the treatment or prevention of muscle, particularly cardiac or skeletal muscle, impairment or fatigue or mitochondrial dysfunction. Liquid compositions for rehydration during or after exercise, comprising water, a sugar carbohydrate and a compound that reduces free fatty acids circulating in blood plasma are also disclosed.

There is a need to improve endurance during exercise and muscle recovery after exercise, particularly intense exercise in healthy people.

We have now surprisingly found that esters of D-β-hydroxybutyrate may reduce muscle or liver glycogen loss and protein loss in a subject during exercise, improve endurance during exercise and muscle recovery after exercise. The ester of D-β-hydroxybutyrate has been found to increase skeletal muscle glucose and glycogen, decrease glycolysis, and alleviate the significant decrease in intramuscular leucine, isoleucine and valine that occurs during exercise, compared to the changes in the same subject after ingestion of a calorifically comparable carbohydrate or fat drink.

In a first aspect, the invention provides the use of an ester of D-β-hydroxybutyrate to improve endurance of a healthy subject during exercise by reducing muscle glycogen loss and/or loss of protein during exercise. The invention also provides the use of an ester of D-β-hydroxybutyrate to aid muscle recovery in a healthy subject after exercise by reducing muscle glycogen loss and/or loss of protein

Reference to reducing glycogen loss or protein loss herein also includes reducing the rate of reduction of glycogen loss or protein loss.

Reference to glycogen breakdown or loss or protein breakdown or loss as employed herein refers to the level of the material as compared to the level of material present without administration of the product of the invention and includes the case where the glycogen or protein breaks down and the product of the invention promotes the production of new glycogen or protein.

The ester of D-β-hydroxybutyrate is also suitable for use in reducing the loss of muscle glycogen and/or loss of protein during exercise.

The ester of D-β-hydroxybutyrate is especially useful in improving endurance during intense exercise and improving muscle recovery after intense exercise, during which glycogen loss and protein loss may typically occur.

Suitably, acute muscle breakdown is reduced in a healthy subject carrying out exercise, especially intense exercise. The term "healthy" as employed herein refers to the general health of the subject being sound and also to the case where the subject may have a condition which may be considered as an impairment to good health or as unhealthy if diagnosed by a physician but which does not materially affect the subjects capability for exercise.

This disclosure provides a method of reducing muscle breakdown during exercise comprising administering the ester of D-β-hydroxybutyrate to a subject susceptible to muscle breakdown to reduce muscle breakdown or to reduce the rate of such breakdown.

As used herein, the term "ketone", "ketone body" or "ketone bodies" means a compound or species which is a ketone or a ketone body precursor, that is, a compound or species which is a precursor to a ketone and which may be converted or metabolised to a ketone. A "ketone body" ester is an ester of such a compound or species.

The ester of D-β-hydroxybutyrate reduces the breakdown of glycogen (glycogen sparing), decreases glycolysis and the build-up of lactate, and decreases protein breakdown, which improves endurance and, in particular, aids muscle recovery following exercise. The ester of D-β-hydroxybutyrate also provides substrate for energy and, with the decrease in muscle breakdown, improves endurance of the subject during exercise. Advantageously, ingestion of an ester of D-β-hydroxybutyrate aids muscle recovery after exercise whereby subjects, particularly elite athletes and other subjects who have undergone intense exercise, may recover more rapidly and completely in a given period of time.

The invention is especially useful in aiding muscle recovery of a healthy person, particularly physically fit subjects, elite athletes, military personnel and the like, for example a person whose blood when tested does not show medical causes which may influence physical performance, for example iron deficiency, haemoglobin (Hb), electrolytes, white cell count and fasting glucose. The invention is particularly useful in aiding recovery in physically fit subjects, particularly in subjects having high levels of fitness, for example athletes and military personnel, particularly elite and high performance athletes where rapid recovery may still be improved.

Suitably, after ingestion of the ester of D-β-hydroxybutyrate, loss of muscle leucine, isoleucine and valine in the subject is reduced by at least 10%, preferably by at least 30% and more preferably at least 50% during exercise compared to the level of leucine, isoleucine and valine in the muscle without administration of the ester of D-β-hydroxybutyrate.

Suitably, after ingestion of the ester of D-β-hydroxybutyrate, loss of muscle or liver glycogen in the subject is reduced by at least 10%, preferably by at least 30% and more preferably at least 50% during exercise compared to the level of glycogen without administration of the ester of D-β-hydroxybutyrate.

Suitably, after ingestion of the ester of D-β-hydroxybutyrate, the rate of loss of glycogen or protein is reduced, preferably by at least 1%, more preferably at least 5%, especially at least 10% during exercise compared to the level of glycogen without administration of the ester of D-β-hydroxybutyrate.

An ester of D-β-hydroxybutyrate may be employed in the invention. Preferably, the ester of D-β-hydroxybutyrate is an ester of a polyhydric alcohol wherein the polyhydric alcohol is not fully esterified.

The esters according to the claims may be derived from alcohols and compounds containing one or more free hydroxyl groups. The-D β-hydroxybutyrate moiety is preferably monomeric. Monoesters are especially preferred and esters where two or more hydroxyl groups have been esterified but the esterified hydroxyl groups are not in a "beta-relationship, in which the hydroxyl groups are not attached to adjacent carbon atoms.

Suitable alcohols include butanediol, especially, 1,3-butanediol, altrose, arabinose, dextrose, erythrose, fructose, galactose, glucose, glycerol, gulose, idose, lactose, lyxose, mannose, ribitol, ribose, ribulose, sucrose, talose, threose, xylitol, xylose. Preferably the alcohol is selected from *R*-1,3-butanediol and glycerol.

Whilst parenteral administration of ketone bodies is known, for example from US-A-6,136,862, oral administration of the ketone body or ketone body ester is desirable in order to rapidly raise circulating ketone levels, which would not be feasible with parental administration or injection due to the volume of salt or acid that might be required. Advantageously, oral administration is more convenient than parenteral administration, the subject is more likely to comply with a dosing regimens, particularly where multiple doses of the ketone are to be ingested over relatively short intervals and where doses are consumed to provide improved athletic performance and possible aversion of the subject to needles may be avoided.

Once ingested, the ketone body then needs to pass from the gut to the blood in order to provide a physiological effect. The concentration of ketone in the blood depends on the level of uptake of the ketone from the gut to the blood. For ketones with a relatively low uptake, a higher level of ketone will be required in the gut. This in turn requires a greater volume of ketone to be ingested to achieve a given blood concentration of ketone.

We have found that a monoester of D-β- hydroxybutyrate with R-1,3 butanediol and/or a monoester of D-β-hydroxybutyrate with glycerol advantageously are less aversive to taste than oligomeric esters and esters of other alcohols. The subject may more readily ingest these esters and adhere to a dosing regimen. Furthermore, these monoesters provide a surprisingly high level of uptake thereby enabling high blood concentrations of hydroxybutyrate to be achieved upon consumption of an oral dose.

In an especially preferred embodiment, the ester according to the claims is a monoester of butane-1,3-diol with D-β-hydroxybutyrate, for example 3-hydroxybutyl-D-β-hydroxybutyrate and a mono ester and a diester of glycerol with D-β-hydroxybutyrate. The ester is preferably in enantiomerically enriched form.

The present disclosure further relates to a method of reducing muscle breakdown by administration of the ester according to the claims in a dose regime wherein the regime comprises administering in at least one dose of the ester to provide a circulating level of hydroxybutyrate and acetoacetate in the blood from 0.1-or 1 to 20 mM, preferably 0.5- or 1 to 10 mM and optimally 1 to 8mM for example 2 to 5 mM wherein at least one dose comprises the ester in an amount of at least 0.1 g/kg bodyweight of the subject per dose and preferably 0.3 to 1.5 g/kg for example at least 0.3 to 0.75 g/kg bodyweight.

The ester of D-β-hydroxybutyrate, preferably (*R*)-3-hydroxybutyl (*R*)-3-hydroxybutyrate, is suitable for use in raising blood D-β-hydroxybutyrate concentration to at least 1 mM, preferably to at least 2 mM, especially 3 mM after oral administration to a subject of monoester at 0.5 g/kg of bodyweight of the subject. Upon oral administration of a dose of *(R)*-3-hydroxybutyl *(R)*-3-hydroxybutyrate of 1 g/kg of body weight of the subject, the blood D-β-hydroxybutyrate concentration is suitably at least 4 mM, preferably at least 5 mM, especially at least 6 mM. Upon oral administration of a dose of *(R)*-3-hydroxybutyl *(R)*-3-hydroxybutyrate of 1.5 g/kg of body weight of the subject, the blood D-β-hydroxybutyrate concentration is suitably at least 7 mM, preferably at least 8 mM, especially at least 9 mM.

Oral administration may be carried out in a single dose or multiple doses. Blood plasma levels of ketone may be determined by commercially available testing kits, for example, Ketostix, available from Bayer, Inc. Breath acetone levels may also be determined using a commercially available kit.

*(R)*-3-hydroxybutyl *(R)*-3-hydroxybutyrate is particularly advantageous as it allows a large rise in blood Dβ-hydroxybutyrate to be achieved with oral ingestion of a much smaller volume of material than with other ketones and other forms of delivery for example parenteral. A subject ingesting the material prior to or during physical exercise is accordingly much more readily able to ingest adequate ketone in order to provide a physiologically beneficial response and aid muscle recovery without risk of physical discomfort either due to a large volume or bitter or otherwise aversive flavour. The high level of blood D-β-hydroxybutyrate concentration is also maintained for a longer period than after other ketones. To maintain raised levels, a lower frequency of administration of further doses is required than for other ketones.

The invention provides in a further embodiment a composition comprising an ester of D-β-hydroxybutyrate as described in relation to the first aspect of the invention .

Suitably the composition comprises water and the claimed ester. Preferably, the composition further comprises a flavouring and optionally one or more of a protein, carbohydrate, sugars, fat, fibre, vitamins and minerals.

Different ketone bodies or ketone body esters have different levels of uptake. We have found that ketone esters are digested more effectively than other forms of ketone for example triolides and oligomers. In order to benefit from the relative ease of digestion, the ketone body is the claimed ketone ester. As a practical benefit, to achieve a given level of plasma ketone, the composition may contain a lower level of the claimed ketone ester than if another ketone body were to be used, so allowing ready ingestion and comfort prior to or during exercise such that decreased loss of muscle glycogen and protein during, and improving endurance during and recovery after, exercise may be secured without discomfort or unpleasantness for the subject in ingesting a material. This is especially beneficial where the level of exercise is vigorous or prolonged. Advantageously, this allows a subject to ingest doses of the ketone body or ketone body ester immediately before, during or after exercise to aid muscle recovery.

Where the the claimed ester is provided as a composition in solid form, the level ester in the composition suitably comprises at least 5% by weight of the hydroxybutyrate ester, more preferably at least 10% by weight and up to 95% by weight of the composition. Whilst a level of 15 to 30% by weight of the dry composition may be suitable, for example where the composition is a dry powder intended for use with a liquid to produce a liquid composition, a solid bar or product form suitably comprises from 30 to 95%, especially 50 to 95% by weight of the composition. Where the composition is in liquid form, the composition suitably comprises the claimed ester at a level of at least 1%, for example 3 to 40% by weight of the liquid composition but may be higher for example up to 90% by weight of the composition depending on whether the composition is intended to be taken as a single dose or in multiple smaller doses to reach the desired blood ketone level.

The composition in liquid form suitably comprises the dry composition diluted with a suitable liquid, for example water, fruit juice or milk, preferably at a ratio of 1:1 to 1:10, more preferably 1:3 to 1:7 of dry composition to liquid. The level of ketone body which is organoleptically acceptable will vary according to the precise composition and its form and the effect of other components of the composition.

The composition may be solid, for example a powder, tablet, bar, confectionary product or a granule and intended for use as a solid oral dose form. In another embodiment, the solid composition may be mixed before use with a liquid, preferably water, fruit based liquid or a dairy product, for example milk and yoghurt, to provide a liquid drink for the user. Milk, fruit juice and water are especially preferred as a carrier for the composition. The composition may be provided, as desired, as a liquid product in a form ready for consumption or as a concentrate or paste suitable for dilution on use. The diluent for use with the liquid composition is preferably milk, fruit juice or water.

When the composition is in solid form the composition may further comprise one or more of the following components:
- a diluent for example lactose, dextrose, saccharose, cellulose, corn starch or potato starch;
- a lubricant for example silica, talc, stearic acid, magnesium or calcium stearate and/or polyethylene glycols;
- a binding agent for example starches, arabic gums, gelatin, methylcellulose, carboxymethylcellulose, or polyvinyl pyrrolidone;
- a disintegrating agent such as starch, alginic acid, alginates or sodium starch glycolate;
- an effervescing agent;
- a dyestuff;
- a sweetener;
- a wetting agent for example lecithin, polysorbates, lauryl sulphates.

The composition may also be provided in encapsulated form provided that the encapsulation material and the quantity in which it is used is suitable for safe human consumption. However, encapsulation is not preferred.

A composition of the invention may contain a medium chain triglyceride (MCT) and optionally their associated fatty acids. MCTs comprise fatty acids with a chain length of between 5 and 12 carbon atoms. It is known that a diet rich in MCT results in high blood ketone levels. Suitable medium chain triglycerides are represented by the following formula CH₂R¹-CHR²-CH₂R³ wherein R1, R2 and R3 are fatty acids having 5 to 12 carbon atoms. Preferably, MCTs wherein R1, R2, and R3 are fatty acids containing a six-carbon backbone (tri-C6:0) are employed as it is reported that tri-C6:0 MCT are absorbed very rapidly by the gastrointestinal track.

Where an MCT is employed, suitably the composition of the invention comprises i) a ketone body, preferably a ketone monoester, more preferably a D β-hydroxybutyrate monoester and ii) an MCT, preferably tri-C6:0 MCT.

The composition of the invention may also comprise L-carnitine or a derivative of L-carnitine. Examples of derivatives of L-carnitine include decanoylcamitine, hexanoylcarnitine, caproylcarnitine, lauroylcarnitine, octanoylcarnitine, stearoylcarnitine, myristoylcarnitine, acetyl-L-carnitine, O-Acetyl-L-carnitine, and palmitoyl-L-carnitine. Where a carnitine is employed, suitably the composition of the invention comprises i) a D-β-hydroxybutyrate ester, preferably D β-hydroxybutyrate monoester and ii) L-carnitine or a derivative of L-carnitine.

In a further embodiment, the composition may comprise i) a D-β-hydroxybutyrate ester, preferably D β-hydroxybutyrate monoester ii) a MCT, preferably tri-C6:0 MCT or a tri-C8:0 MCT and iii) L-carnitine or a derivative of L-carnitine.

Where MCT and L-carnitine or its derivative is employed, suitably the MCT is emulsified with the carnitine. Preferably 10 to 500 g of emulsified MCT is combined with 10 to 2000 mg of carnitine for example 50 g MCT (95% triC8:0) emulsified with 50 g of mono- and di-glycerides combined with 500 mg of L-carnitine.

The MCT may be present in a greater amount than the ester but preferably the level of the ester is greater than the level of the MCT.

The composition may be in the form of a solid or in the form of a liquid composition or a gel. Suitable solid forms of the composition include a bar or powder suitable for mixing with a liquid, for example water, milk or fruit juice at the point of use. Suitable forms of liquid composition include for example a syrup, an emulsion and a suspension. Suitably, in the form of a syrup, the composition further may contain as carrier, for example, saccharose or saccharose with glycerol and/or mannitol and/or sorbitol. In the form of a suspension or emulsion, the composition may contain as a carrier, for example, a natural gum, agar, sodium alginate, pectin, methylcellulose, carboxymethylcellulose or polyvinyl alcohol.

The composition may also be a food product, food supplement, dietary supplement, functional food or a nutraceutical or a component thereof.

A food product is an edible material composed primarily of one or more of the macronutrients protein, carbohydrate and fat, which is used in the body of an organism to sustain growth, repair breakdown, aid vital processes or furnish energy. A food product may also contain one or more micronutrients such as vitamins or minerals, or additional dietary ingredients such as flavourants and colourants. The term food product as used herein also covers a beverage.

Examples of food products into which the composition may be incorporated as an additive include snack bars, cereals, confectionery and probiotic formulations including yoghurts. Examples of beverages include soft beverages, alcoholic beverages, energy beverages, dry drink mixes, nutritional beverages and herbal teas for infusion or herbal blends for decoction in water.

A nutraceutical is a food ingredient, food supplement or food product which is considered to provide a medical or health benefit, including the prevention and treatment of disease. In general a nutraceutical is specifically adapted to confer a particular health benefit on the consumer. A nutraceutical typically comprises a micronutrient such as a vitamin, mineral, herb or phytochemical at a higher level than would be found in a corresponding regular food product. That level is typically selected to optimise the intended health benefit of the nutraceutical when taken either as a single serving or as part of a diet regimen or course of nutritional therapy.

A functional food is a food that is marketed as providing a health benefit beyond that of supplying pure nutrition to the consumer. A functional food typically incorporates an ingredient such as a micronutrient as mentioned above, which confers a specific medical or physiological benefit other than a nutritional effect. A functional food typically carries a health claim on the packaging.

A kit is provided comprising a product selected from an ester of D-β-hydroxybutyrate and a composition according to the invention and a ketone monitor and optionally instructions as to the level of product to consume per unit body weight to achieve a pre-determined level of blood plasma ketone and a dosage regimen to maintain blood plasma ketone at the pre-determined level to reduce muscle breakdown. The user suitably consumes the product and may then periodically test their breath or blood plasma ketone level to determine whether further ingestion of ketone ester is required to reach or to maintain the desired blood plasma ketone level.

Suitably the claimed ester or composition comprising it is provided with instructions for consumption. Suitably the instructions to consume one or more doses of a ketone body ester or composition according to the invention per day or consume a dose prior to exercise, preferably at least 10 minutes, more preferably at 30 minutes and at most 1 hour prior to exercise.

For prolonged exercise, for example more than 20 minutes, 2 or more doses, more preferably 2 to 8 doses for example 3 or 6 doses may be consumed periodically to raise or to maintain raised blood plasma ketone levels. Suitably the doses are consumed at regular intervals as this maintains a more even level of blood ketone content although a user may consume a dose to maintain or improve power output so as to "prime" the blood with ketone.

The invention is described by reference to the following examples

### Example 1

Comparison of Ketone Ester with Carbohydrate and Fat-Based Nutritional Drinks Plasma metabolites and skeletal muscle metabolism in high performance athletes (n = 10) were measured after providing carbohydrate, long chain fat or ketone drinks before and during cycling exercise.

### Methods

Cycling exercises were carried out to illustrate the benefits of the invention in the form of a drink, vs. control drinks, regarding muscle endurance during exercise and muscle recovery after exercise.

*The Athletes:* Ten male high performance athletes from endurance sports were recruited to take part in this study. All had international competitive experience; several had represented Great Britain at world championship level and were healthy. They were asked not to perform strenuous exercise within 48 hours of the trials, to refrain from alcohol and caffeine for 24 hours and to consume an identical pre-testing meal the night before every visit. The athletes presented following an overnight fast and testing was at the same time of day (starting at 0800 h) to minimise the impact of diurnal patterns on performance. Athletes repeated their preparatory routines in identical fashion prior to all tests. Athletes were tested within the same macrocycle of training and at the same time within a training week.

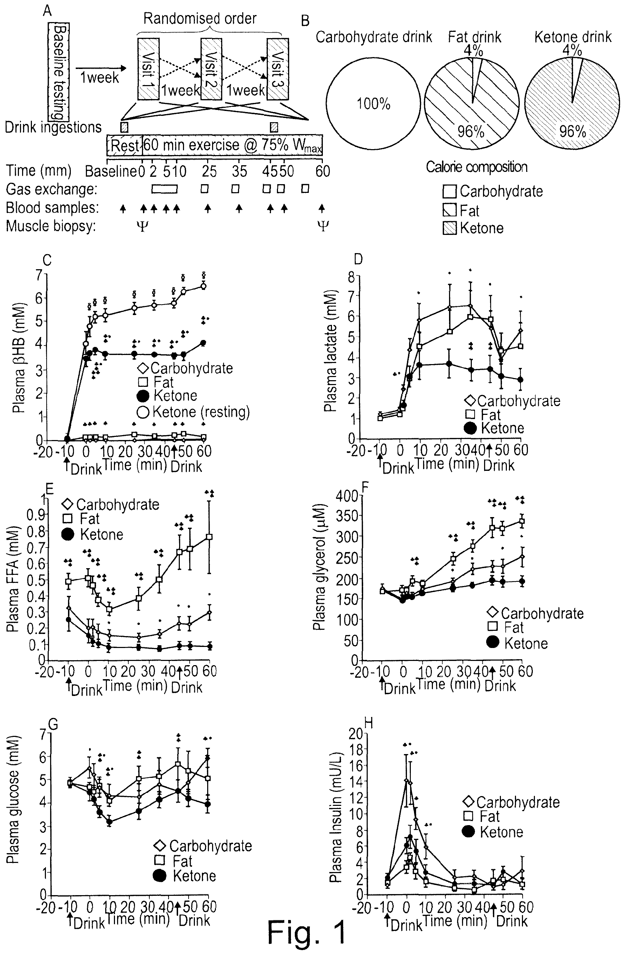

*The Cycling Test:* All participants undertook an incremental (25W/3 min) exercise test to exhaustion in which the wattage was increased every 3 minutes on an electronically braked bicycle ergometer (Monark 928e, Sweden) for the determination of VO_{2 Max} and W_{Max} at least 1 week prior to the first trial. The same ergometer was used for subsequent exercise tests, which were completed by all athletes. Each athlete completed three experimental trials consisting of 60 min of constant load cycling at 75% of W_{Max} performed in a randomised, single-blind, cross-over manner, with 1 week between trials (Figure 1A).

*Drinks preparation:* Drinks were isocaloric in energy (mean calorific value 257 ± 15 Kcal) and taste matched using sweeteners (Neotame™, NutraSweet, USA) or bitter additive (Symrise, product number 648352, UK) to ensure blinding. Substrate calories were bodyweight-adjusted, and dosed to ensure a minimum carbohydrate delivery of 1.2 g/min of exercise when drinking the carbohydrate drink. Drinks were made up from commercially available sports water (Glaceau, UK), and matched for tonicity (13% solutions for all arms). As shown in Figure 1B all drinks contained a minimum of 96% of their energy from a sole substrate, as carbohydrate (Maltodextrin, fructose ratio 5:1, Gu Gels, Berkeley, USA), long chain triglyceride (Calogen™, Zoetermeer, Netherlands), or (R)-3-hydroxybutyl (R)-3-hydroxybutyrate , a ketone ester according to the invention. For the tests the drink was administered at 3.5 ml/ kg body weight containing 573 mg/kg body weight of the substrate (ketone ester, fat or carbohydrate).

Subjects ingested a 75% dose of the drink containing carbohydrate, emulsified fat, or ketone ester 15 min prior to the start of exercise, over a 5 min time interval. At 45 min, athletes were stopped for 1 min and asked to ingest the second, smaller volume (25% dose) of drink as a 'top up' for the remaining 15 min of exercise. During the trial, subjects were allowed water *ad libitum.*

*Allocation of drinks:* Drinks according to the invention and control drinks were prepared as set out below. Allocation of the drinks to the participant athletes was concealed and the trials were randomized, single-blind, cross-over and placebo-controlled. A double randomization method was used; the order of drink allocation was determined using a random number generator only when the participant came in for each trial, and the order of participation was determined by participant availability. The drinks of the invention and control were indistinguishable in that they had the same taste, appearance and volume for each athlete and were randomly assigned. On questioning, the athletes did not know which drink they had consumed.

*Parameters Measured:* Various measurements were made on athletes were measured using blood tests and muscle biopsies, from which metabolites were extracted and analysed employing the following procedures:
*Blood sampling:* During exercise, blood samples (2-3 ml) were obtained via a 22 G venous catheter inserted percutaneously into an antecubital vein. A three-way tap was used to allow repeated sampling. Samples were immediately stored on ice, centrifuged (3600 rpm for 9 min) and subsequently stored at -80°C until further analysis. Glucose, free fatty acids, triglycerides, D-β-hydroxybutyrate and lactate were assayed using a commercial automated bench-top analyzer (ABX Pentra, Montpellier, France). Glycerol and insulin assays were performed using Elisa kits (Mercodia, Uppsala, Sweden).

*Muscle biopsies:* Muscle tissue was collected using percutaneous needle biopsies from the lower third of the Vastus Lateralis muscle with a biopsy gun (Bard Monopty™, Bard Biopsy Systems, USA). Samples were obtained from new incisions before and immediately after each exercise bout. Tissue was rapidly frozen in liquid nitrogen and stored at -80 °C until further analysis.

*Metabolite extraction from skeletal muscle:* Metabolites were double-extracted from approximately 100 mg tissue. The aqueous and organic fractions were separated and further split into 2 identical volumes to allow multiple analyses.

*¹H-NMR analysis of aqueous metabolites:* Half of the aqueous fraction (-25 mg wet weight tissue) was dried under nitrogen, and resuspended in 600 µL D₂O containing 0.09% w/v NaCl (Sigma), 0.01% w/v NaN₃ (Sigma) and 0.25 mM deuterated sodium-3-trimethylsilylproprionate (NaTMSP-2,2,3,3-D4, Cambridge Isotope Laboratories, Inc.) as a chemical shift reference. Samples were analysed on a Bruker NMR spectrometer interfaced with an 11.8 Tesla superconducting magnet at 310K using a ¹H-NOESY 1D pulse sequence with 128 scans. Data were integrated using fixed integral sizes of 0.02 ppm within 1D Spec Manager (v12, Advanced Chemistry Development, Inc.).

*Statistics:* Results are expressed as means ± SEM and significance was taken at p < 0.05. Although one subject declined to have skeletal muscle biopsies, all clinical and laboratory data were analysed for all subjects (no attrition or exclusions). Statistical analysis was performed using SPSS (V17, Chicago, USA). Blood metabolites were compared between arms using one way ANOVA with Tukey post hoc correction.

### Results

Ingestion of a drink (3.5 ml/ kg body weight) containing 573 mg/kg body weight of ketone ester resulted in a rapid rise in circulating D-β-hydroxybutyrate from overnight fasted levels of 0.13 ± 0.03 mM to 3.5 ± 0.3 mM during 10 min of rest, where they remained throughout 60 min of exercise as shown in Figure 1C. When athletes remained resting, plasma D-β-hydroxybutyrate concentrations increased to > 5 mM. D-β-hydroxybutyrate concentrations after the ketone drink were higher than concentrations observed following drinks containing carbohydrate or long chain fats.

Lactate concentrations were the same at baseline for all drinks (Figure 1D). However, after the onset of exercise, blood lactate concentrations were significantly lower after the ketone drink than after carbohydrate or fat drinks, resulting in average lactate concentrations ∼2-3 mM (∼50%) lower than with the carbohydrate drink throughout the cycle and lower than the fat drink at 30 and 45 min.

Free fatty acid (FFA) concentrations were significantly higher at baseline after the high-fat low-carbohydrate intake (Fig. 1E) and remained elevated throughout exercise compared with carbohydrate or ketone drinks, reaching ∼0.8 mM at the end of exercise. FFA concentrations were the same at baseline and fell after carbohydrate and ketone drinks. Ketosis significantly suppressed the rise in FFA seen after 25 min of exercise compared with both fat and, to a lesser extent, carbohydrate drinks. Exercise caused significant increases in plasma glycerol following both the carbohydrate and fat drinks (Fig. 1F), but not after the ketone drink.

Blood glucose concentrations were the same for all athletes at baseline, but increased significantly within 10 min of the carbohydrate drink (Fig. 1G). Blood glucose fell during the first 10 min of exercise after the carbohydrate or ketone drinks, and was significantly lower after the ketone drink than after the fat or carbohydrate drinks within 5 min of exercise, remaining lower than the fat drink for much of the cycle.

Plasma insulin concentrations were significantly elevated following the carbohydrate drink compared with the fat and ketone drinks (Fig. 1H). Insulin concentrations peaked 10 min after the carbohydrate drink, and fell to baseline levels after 25 min of exercise. There were no significant differences in insulin after the fat and ketone drinks.

Following the ketone drink, the lower plasma lactate, FFA and glycerol levels during exercise suggested that the exercise could have been continued for longer, in other words, the ketosis improved endurance.

### Diet altered skeletal muscle metabolites during exercise

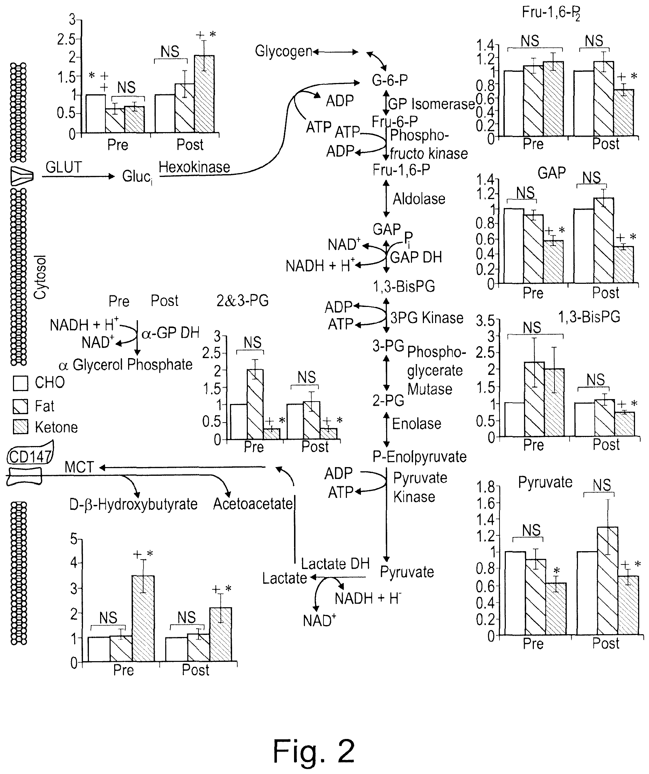

D-β-hydroxybutyrate and other metabolites were measured in skeletal muscle biopsies before and after the cycling exercise (Figure 2). At rest, 10 min after the ketone drink, intramuscular concentrations of D-β-hydroxybutyrate were ∼3 fold higher than after the ingestion of carbohydrate or fat drinks and remained raised at the end of exercise, double the concentrations following either fat or carbohydrate drinks. Thus, the increase in plasma D-β-hydroxybutyrate was reflected in the increase in intramuscular D-β-hydroxybutyrate. Intramuscular glucose was increased pre-exercise following the carbohydrate drink compared with fat and ketone drinks and was significantly elevated following the ketone drink plus exercise. Pre-exercise muscle concentrations of the glycolytic intermediates, glyceraldehyde-3-phosphate, 2&3-phosphoglycerate and pyruvate, were significantly lower following the ketone drink compared with carbohydrate and fat drinks. Fructose-1,6-bisphosphate, dihydroxyacetone phosphate (DHAP), and 1,3-bisphosphoglycerate were the same at rest in all subjects.

Following exercise, and with the exception of DHAP, concentrations of all muscle glycolytic intermediates were significantly lower after the ketone drink compared with carbohydrate and fat drinks, suggesting that ketosis suppressed skeletal muscle glycolysis and explaining the 2-3 mM lower blood lactate concentration described above. Glycolytic intermediates were the same following fat and carbohydrate drinks.

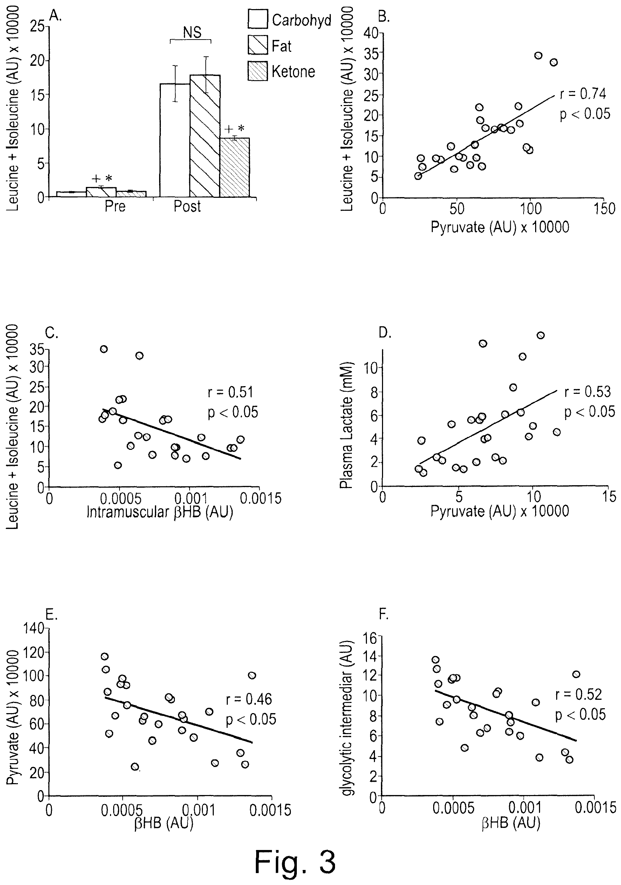

Branched chain amino acids (BCAA), leucine, isoleucine and valine, are mobilised during exercise as muscle energetic and anapleurotic demands increase. At rest, skeletal muscle BCAAs were significantly higher after the fat drink than after carbohydrate or ketone drinks as shown in Figure 3A. During exercise, leucine, isoleucine and valine increased due to muscle catabolism, but were 50% lower following the ketone drink than carbohydrate or fat drinks as shown in the post-exercise data in Figure 3A. The exercise-induced demand for anapleurotic substrates was reflected in the strong positive relationship between muscle leucine, isoleucine and valine and muscle pyruvate as shown in Figure 3B. Figure 3C shows that the higher the intracellular beta-hydroxybutyrate (□HB) levels, the lower the breakdown of muscle to make glucose, and the lower the flux through glycolysis (and pyruvate), the □HB providing a source of energy. Plasma lactate correlated positively with muscle pyruvate (Figure 3D), supporting the conclusion that the lower lactate during ketosis was due to decreased muscle glycolysis. Figure 3E shows that by increasing intramuscular D-β-hydroxybutyrate during exercise there is a reduced glycolytic demand as shown by decreased leucine, isoleucine and valine, pyruvate and the sum of the glycolytic intermediates (Figure 3F). Lower levels of leucine, isoleucine and valine indicate a decrease in the use of glucose during exercise.

### Conclusions

Administration of a dietary ketone ester to elevate circulating ketones, according to the invention, significantly altered plasma substrates in athletes and reduced muscle glycogen and protein breakdown, and improved endurance during and recovery after exercise.

The results suggest that ketone metabolism may hold hierarchical preference over carbohydrate and fat metabolism even during conditions that traditionally strongly favour carbohydrate oxidation, such as intense exercise. Intra-muscular D-β-hydroxybutyrate levels were ∼3-fold higher following the ketone drink, which decreased glycolytic intermediates, whilst sustaining TCA cycle metabolites during the same exercise, suggesting that ketones were oxidised preferentially as an alternative to pyruvate.

Exercise provides a model of physiological stress and has significant parallels with diseases in which energetic demands are high and switches in substrate selection may occur

### Example 3

### Ketosis reduced

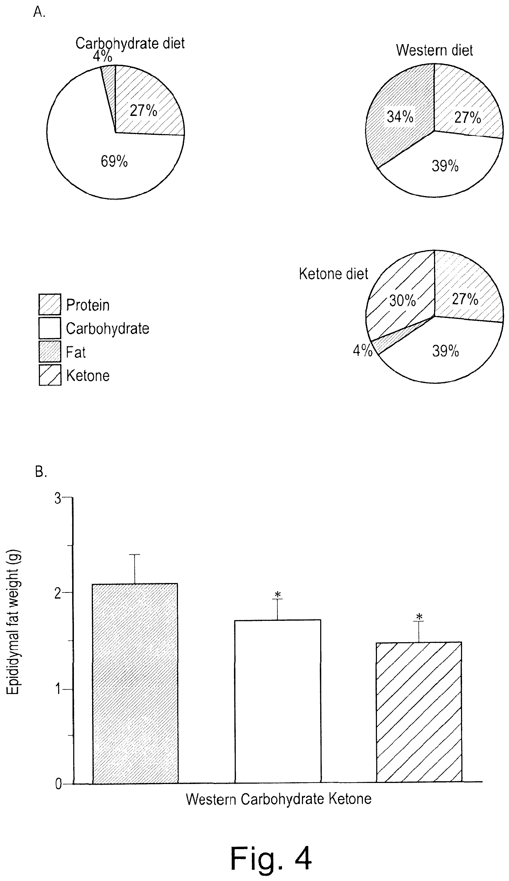

Adult male Wistar rats (n = 65) were fed standard laboratory chow *ad libitum* prior to starting the experimental diets. Rats were fed one of three isocaloric diets: (a) a fat-rich "Western" diet in which 30% of kcal came from added palm oil, (b) a high-carbohydrate diet, in which 30% of kcal came from added corn starch, or (c) a ketone diet, in which 30% of kcal came from monoester of D β hydroxybutyrate-R 1,3 butanediol monoester, a ketone ester according to the invention, as shown in Figure 4A. Each diet contained 1.76 kcal/g and rats were pair-fed for 66 days.

Rats on the ketone diet had the same body weight, but epididymal fat weights were 35% lower (Fig. 4B) than in those fed the Western diet. The markedly less fat, and therefore more muscle, was due to reduced muscle breakdown over the 66 days.

Nutritional ketosis advantageously elevated circulating D-β-hydroxybutyrate without the negative effects of a high-fat ketogenic diet or ketone acid/salt infusions, and would be beneficial for endurance during exercise and recovery following exercise in healthy subjects.

## Claims

1. Use of an ester of D-β-hydroxybutyrate to improve endurance of a healthy subject during exercise by reducing muscle glycogen loss and/or loss of protein during exercise.

2. Use of an ester of D-β-hydroxybutyrate to aid muscle recovery in a healthy subject after exercise by reducing muscle glycogen loss and/or loss of protein.

3. Use according to claim 1 or claim 2 wherein the ester of D-β-hydroxybutyrate comprises an ester of a polyhydric alcohol wherein the polyhydric alcohol is not fully esterified.

4. Use according to claim 3 wherein the polyhydric alcohol is selected from R 1,3 butanediol or glycerol.

5. Use according to any one of the preceding claims wherein the ester of D-β-hydroxybutyrate comprises a monoester.

6. Use according to claim 5 wherein the monoester comprises a monoester of D-β-hydroxybutyrate with R-1,3-butanediol and/or a monoester of D-β-hydroxybutyrate with glycerol.

7. Use of a composition comprising i) an ester of D-β-hydroxybutyrate and ii) a mid-chain triglyceride to improve endurance of a healthy subject during exercise by reducing muscle glycogen loss and/or loss of protein during exercise.

8. Use of a composition comprising i) an ester of D-β-hydroxybutyrate and ii) a mid-chain triglyceride to aid muscle recovery in a healthy subject after exercise by reducing muscle glycogen loss and/or loss of protein.

9. Use according to claim 7 or claim 8 wherein the mid-chain triglyceride comprises a fatty acid with a chain length 5 to 12 carbon atoms.

10. Use according to any one of claims 7 to 9 wherein the mid-chain triglyceride comprises a compound of formula CH₂R¹-CHR²-CH₂R³ wherein R1, R2 and R3 are fatty acids having 6 carbon atoms.

## Patentansprüche

1. Verwendung eines Esters von D-β-Hydroxybutyrat zur Verbesserung der Ausdauer einer gesunden Person bei Übungen durch Verringerung des Muskel - Glykogenverlustes und/oder Proteinverlustes bei Übungen.

2. Verwendung eines Esters von D-β-Hydroxybutyrat zur Unterstützung der Muskelregeneration in einer gesunden Person nach Übungen durch Verringerung des Muskel - Glykogenverlustes und/oder Proteinverlustes.

3. Verwendung gemäß Anspruch 1 oder Anspruch 2, wobei der Ester von D-β-Hydroxybutyrat einen Ester eines mehrwertigen Alkohols umfasst, wobei der mehrwertige Alkohol nicht vollständig verestert ist.

4. Verwendung gemäß Anspruch 3, wobei der mehrwertige Alkohol aus (R)-1,3-Butandiol oder Glycerol ausgewählt ist.

5. Verwendung gemäß einem der vorhergehenden Ansprüche, wobei der Ester von D-β-Hydroxybutyrat einen Monoester umfasst.

6. Verwendung gemäß Anspruch 5, wobei der Monoester einen Monoester von D-β-Hydroxybutyrat mit (R)-1,3-Butandiol und/oder einen Monoester von D-β-Hydroxybutyrat mit Glycerol umfasst.

7. Verwendung einer Zusammensetzung, umfassend i) einen Ester von D-β-Hydroxybutyrat und ii) ein mittelkettiges Triglycerid zur Verbesserung der Ausdauer einer gesunden Person bei Übungen durch Verringerung des Muskel - Glykogenverlustes und/oder Proteinverlustes bei Übungen.

8. Verwendung einer Zusammensetzung, umfassend i) einen Ester von D-β-Hydroxybutyrat und ii) ein mittelkettiges Triglycerid zur Unterstützung der Muskelregeneration in einer gesunden Person nach Übungen durch Verringerung des Muskel - Glykogenverlustes und/oder Proteinverlustes.

9. Verwendung gemäß Anspruch 7 oder Anspruch 8, wobei das mittelkettige Triglycerid eine Fettsäure mit einer Kettenlänge von 5 bis 12 Kohlenstoffatomen umfasst.

10. Verwendung gemäß einem der Ansprüche 7 bis 9, wobei das mittelkettige Triglycerid eine Verbindung der Formel CH₂R¹-CHR²-CH₂R³ umfasst, wobei R¹, R² und R³ Fettsäuren mit 6 Kohlenstoffatomen sind.

## Revendications

1. Utilisation d'un ester de D-β-hydroxybutyrate pour améliorer l'endurance d'un sujet sain durant l'exercice par réduction de la perte de glycogène musculaire et/ou de la perte de protéines durant l'exercice.

2. Utilisation d'un ester de D-β-hydroxybutyrate pour aider la récupération musculaire chez un sujet sain après l'exercice par réduction de la perte de glycogène musculaire et/ou de la perte de protéines.

3. Utilisation selon la revendication 1 ou la revendication 2 dans laquelle l'ester de D-β-hydroxybutyrate comprend un ester d'un alcool polyhydrique dans lequel l'alcool polyhydrique n'est pas totalement estérifié.

4. Utilisation selon la revendication 3 dans laquelle l'alcool polyhydrique est sélectionné parmi le R-1,3-butanediol ou le glycérol.

5. Utilisation selon une quelconque des revendications précédentes dans laquelle l'ester de D-β-hydroxybutyrate comprend un monoester.

6. Utilisation selon la revendication 5 dans laquelle le monoester comprend un monoester de D-β-hydroxybutyrate avec du R-1,3-butanediol et/ou un monoester de D-β-hydroxybutyrate avec du glycérol.

7. Utilisation d'une composition comprenant i) un ester de D-β-hydroxybutyrate et ii) un triglycéride à chaîne moyenne pour améliorer l'endurance d'un sujet sain durant l'exercice par réduction de la perte de glycogène musculaire et/ou de la perte de protéines durant l'exercice.

8. Utilisation d'une composition comprenant i) un ester de D-β-hydroxybutyrate et ii) un triglycéride à chaîne moyenne pour aider la récupération musculaire chez un sujet sain après l'exercice par réduction de la perte de glycogène musculaire et/ou de la perte de protéines.

9. Utilisation selon la revendication 7 ou la revendication 8 dans laquelle le triglycéride à chaîne moyenne comprend un acide gras avec une longueur de chaîne de 5 à 12 atomes de carbone.

10. Utilisation selon l'une quelconque des revendications 7 à 9 dans laquelle le triglycéride à chaîne moyenne comprend un composé de formule CH₂R¹-CHR²-CH₂R³ dans laquelle R1, R2 et R3 sont des acides gras ayant 6 atomes de carbone.
